# EUROPÄISCHE PATENTSCHRIFT

(11) **EP 1 500 646 B1**
(45) Veröffentlichungstag und Bekanntmachung des Hinweises auf die Patenterteilung: **09.01.2008**
(21) Anmeldenummer: 04017387.4
(22) Anmeldetag: 22.07.2004
(51) Int. Cl.: C07D 201/12, C07D 201/16

(54) **Verfahren zur Reinigung von Caprolactam aus polyamidhaltigen Abfällen mittels UV-Bestrahlung**
Process for the purification of caprolactam from polyamid containing wastes by UV irradiation
Purification de caprolactam provenant des dechets de polyamides par irradiation UV

(30) Priorität: 23.07.2003 DE 10333539
(43) Veröffentlichungstag der Anmeldung: 26.01.2005
(73) Patentinhaber: Lurgi Zimmer GmbH, 60295 Frankfurt am Main (DE)
(72) Erfinder: Kämpf, Rudolf, Dr., 63584 Haingründau (DE); Wolf, Reinhard, 63517 Rodenbach (DE); Seelig, Joachim, Dr., 63599 Biebergemünd (DE)
(74) Vertreter: Grünecker, Kinkeldey, Stockmair & Schwanhäusser Anwaltssozietät

(56) Entgegenhaltungen:
- DE-B- 1 105 420
- DE-C- 889 199
- US-A- 5 637 700
- US-B1- 6 187 917
- US-B2- 6 579 979
- PATENT ABSTRACTS OF JAPAN vol. 1996, no. 08, 30. August 1996 (1996-08-30) -& JP 08 099954 A (TORAY IND INC), 16. April 1996 (1996-04-16)

## Beschreibung

Die vorliegende Erfindung betrifft ein gegebenenfalls kontinuierliches und wirtschaftliches Verfahren zur Reinigung von Caprolactam aus Polyamidabfällen, geeignet für die Polykondensation zu Polyamid 6. Insbesondere betrifft die vorliegende Erfindung ein Verfahren zum kontinuierlichen Herstellen von Caprolactam aus Polyamid-haltigen Abfällen unter Bestrahlen des Caprolactam-Rohmaterials mit UV-Strahlung.

Bei der Aufbereitung von recycelten Gebrauchsartikeln, die schon jahrelang benutzt wurden, oder Produkten, die aus einer Kunststoffsammlung stammen, lässt sich ein hochwertiges Caprolactam nur mit hohem technischem, energetischem und kostenmäßigem Aufwand erzeugen. So sind Verfahren bekannt, mit deren Hilfe es möglich ist, durch Sortier-, Aufbereitungs- und Reinigungsvorgänge ein reines Caprolactam aus Polyamidabfällen zu erhalten. Die dort beschriebenen Schritte insbesondere die Zugabe von Kaliumpermanganat, wie auch in den US-PSen 6,187,917, 2,752,336, 4,148,792 und 5,637,700 beschrieben, gelten als unabdingbar zum Erreichen einer guten Farbzahl und eines hochwertigen und farbreinen Caprolactams. Da Kunststoffprodukte immer dem Einsatzgebiet angepasst werden, enthalten sie daher Hersteller- und Herstellungs-bedingt Zuschlagsstoffe, Farbmittel, Stabilisatoren, Glasfasern, etc., die eine Herstellung von Caprolactam mit guter Farbzahl erschweren. Daher führt bei einer wirtschaftlichen Wiederverwendung von Polyamidabfällen kein Weg an der Zerlegung in die Monomere vorbei, soll ein Produkt erzeugt werden, das sich von Ware die aus Monomeren der Syntheseroute hergestellt wurde, nicht unterscheidet.

Es ist außerdem bekannt, ein Caprolactam-Wasser-Gemisch, das nach der Depolymerisation erhalten wird, durch fraktionierte Destillation aufzukonzentrieren, wobei es sich im Hinblick auf die Reinheit des Caprolactams wiederum als vorteilhaft erwiesen, Nebenbestandteile kontinuierlich und durch Analysen und online Messungen zu überwachen und die Betriebsbedingungen wie beispielsweise Temperatur, Druck und Rücklaufverhältnis so zu steuern, dass die bestmögliche Entfernung der unerwünschten Nebenprodukte erreicht wird. Trotz der geschilderten weiteren Maßnahmen, muss Kaliumpermanganat zur Verbesserung der Farbe und des Doppelbindungsgehaltes hinzugefügt werden. Diese Methode erfordert daher ebenfalls nachteilig eine Abtrennung des entstandenen Braunsteins in Abscheide- und Filterstufen um eine die Farbreinheit und Feststofffreiheit zu erhalten.

Aus all den vorweg aufgeführten Gründen ist es verständlich, dass es herkömmlicherweise für die Aufbereitung eines aus einer Recyclinganlage gewonnen Rohcaprolactams zu einem Produkt mit guten Farbwerten chemischer Zusatzstoffe bedurfte. In der Praxis hat sich die Zugabe von Permanganat als vorteilhaft herausgestellt, das als ein mildes Oxidationsmittel wirkt und das farbbringende Doppelbindungen, Aldehyde oder andere durch Decarboxylierung entstandene Produkte unter Entstehung von Braunstein abbaut. Nachteilig ist allerdings die Abtrennung des entstandenen Braunsteins durch Agglomerations- und Filtrationsaufwand und die Entsorgung des metallsalzhaltigen Feststoffes.

Weiterhin sind Verfahren bekannt, bei denen Wasserstoffperoxid, Ozon oder Ozon/Sauerstoffmischungen eingesetzt wurden. Diese haben den Vorteil keine Rückstände zu hinterlassen, sind aber aufgrund ihrer hohen Reaktivität von Wasserstoffperoxid, Ozon oder Ozon/Sauerstoffgemisch schwer zu dosieren. Eine Überdosierung führt jedoch zu einer Verschlechterung der Produktqualität des Caprolactams.

DE1105420 beschreibt die Reinigung von vorgereinigtem epsilon-Caprolactam mit UV-Strahlung.

Aus JP08099954 ist die Verwendung von UV-Strahlung zur Reinigung octahydrophenazinhaltiger Caprolactamströme bekannt.

Daher liegt der vorliegenden Erfindung die Aufgabe zugrunde, ein Verfahren zur Verfügung zu stellen, bei dem die Stoffe, die die verminderte Farbqualität im Rohcaprolactam aus einer Polyamidwiederaufbereitung verursachen, entfernt werden, ohne dass Chemikalien verbraucht oder deren Rückstände ausgeschleust werden müssen.

Die Lösung der Aufgabe ist ein Verfahren zum, bevorzugt kontinuierlichen, Herstellen von Caprolactam aus Polyamid-haltigen Abfällen, umfassend
a) Depolymerisieren der Polyamid-haltigen Abfälle, wodurch ein Caprolactam-Rohmaterial und gegebenenfalls ein Nebenbestandteile bzw. Zuschlagsstoffe enthaltender Strom erhalten wird, und
b) Bestrahlen des Caprolactam-Rohmaterials mit UV-Strahlung.

Die im erfindungsgemäßen Verfahren verwendbaren Polyamid-haltigen Abfälle sind ausgewählt aus gebrauchten, verschmutzten oder dem Recyclingkreislauf entnommenen Polyamid 6 Abfällen. Die Polyamid-haltigen Abfälle sind insbesondere bevorzugt ausgewählt aus der Gruppe, bestehend aus Polyamid-haltigen Formkörpern, wie Teilen für Fahrzeuge, Polyamid-haltigen Spritzgussteilen mit Glasfasern und anderen Zuschlägen sowie Polyamid-haltigen Fasern, Teppichen, Teppichbodenbelägen und anderen Polyamid-haltigen Gegenständen des täglichen Lebens, wie Kleidung. Das Polyamid-haltige Material, das der Depolymerisation zugeführt wird, kann außerdem Nichtpolymere und artfremde Zuschlagsstoffe enthalten.

Vor dem Depolymerisieren in Stufe a) des erfindungsgemäßen Verfahrens können die Polyamid-haltigen Abfälle vorzugsweise zunächst sortiert werden, so dass diese überwiegend Polyamid 6 enthalten und die übrigen anderen Stoffe abgetrennt werden.

Caprolactam ist das Monomer das benutzt wird, um Polyamid 6 herzustellen, aus dem dann Teile für Fahrzeuge, Spritzgussteile mit Glasfasern und anderen Zuschlägen, Fasern für Garne aus denen Teppiche, Teppichbodenbeläge oder andere Gegenstände des täglichen Lebens gefertigt werden. Ein großer Teil dieses Materials wird nach dem bestimmungsgemäßen Gebrauch entsorgt und nur ein geringer Teil wird dem Kunststoffkreislauf und der Wiederverwertung zugeführt. Die Polyamid 6 Produkte sind meist nicht die einzigen Kunststoffmaterialien, die aus amidische Gruppen bestehen, und die in einer Sammlung enthalten sind. Es sind auch noch andere Polyamide anzutreffen, die aus unterschiedlichen Disäuren und Diaminen gebildet wurden. Daher wird vor dem Depolymerisieren vorzugsweise eine Kennung und Sortierung in die einzelnen Polyamid-Produktgruppen durchgeführt.

Die Aufbereitung von Polyamid 6 Abfällen nach ihrem bestimmungsgemäßen Gebrauch kann durchgeführt werden, wie in verschiedenen Patenten, beispielsweise DE 197 19 734, EP 0 681 896, US 5,598,980, DE 241 65 73, DE 250 77 44 beschrieben, und umfasst die Erkennung und Sortierung von Nichtpolyamid 6 -Ware, deren Zerkleinerung und die Abscheidung von Schmutz- und Fremdstoffbeigaben. Für die Trennung von einzelnen Komponenten, wie sie beispielsweise bei der Zerkleinerung von Teppichen in Form von Schaumrücken, Polfaser und Träger anfallen, werden bevorzugt mehrstufige Schwimm-/Sinkverfahren benutzt, in deren Folge mittels Zentrifugen und Salzlösungen unterschiedlicher Dichte ein Aufschwimmen der leichteren Fraktion, wie Polypropylengewebe, und ein Absinken von schwererem, gefülltem Teppichrücken bewirkt wird. Eine nach diesem Verfahren gewonnene Polyamid 6 Fraktion wird durch Waschen von anhängender Salzfracht befreit und in einem kontinuierlichen Verfahren getrocknet und über einen Extruder der Depolymerisation zugeführt.

In der Depolymerisation erfolgt beispielsweise nach den aus DE 88 71 99, EP 0 875 504, DE 91 00 56, US 4,605,762 und/oder JP 53-13636 bekannten Verfahren mittels Phosphorsäure eine Spaltung der Polymerketten und eingeleiteter Dampf treibt das entstandene Caprolactam als Wasserdampfgemisch ab.

Das Depolymerisieren in Stufe a) des erfindungsgemäßen Verfahrens wird vorzugsweise bei einer Temperatur von etwa 180 bis etwa 300 °C und Drücken von etwa 0,5 bis etwa 2 bar durchgeführt. Außerdem wird bevorzugt ein basischer oder saurer Katalysator, wie Phosphorsäure, zugesetzt. Die Reaktionsbedingungen während der Depolymerisation werden bevorzugt der sich wandelnden Zusammensetzung des dem Verfahren zugeführten Rohstoffes, nämlich des Polyamid-haltigen Abfalls, angepasst. So kann die Dampftemperatur erhöht, größere Dampfmengen verwendet, das Molverhältnis von Phosphorsäure zu Polyamid verändert und/oder der Druck erhöht oder erniedrigt werden.

Das in Stufe b) mit UV-Strahlung bestrahlte Caprolactam-Rohmaterial ist das nach der Depolymerisation in Stufe a) erhaltene Caprolactam-Rohmaterial. Dieses ist vorzugsweise eine Caprolactam-Lösung, insbesondere eine wässrige Caprolactam-Lösung mit einem Gehalt an Caprolactam zwischen 5 Massen% -und 99 Massen%, vorteilhaft 25 Massen% bis 90 Massen%.

Die UV-Bestrahlung in Stufe b) des erfindungsgemäßen Verfahrens kann mit jedem bekannten UV-Strahler durchgeführt werden. Bevorzugt wird eine Tauchsonde mit UV-Strahler in das Caprolactam-Rohmaterial eingetaucht, insbesondere in einem Reaktor, in dem die Tauchsonde angeordnet ist und diese von dem Caprolactam-Rohmaterial umströmt ist. Bevorzugt liegt die Durchflussgeschwindigkeit der wässrigen Caprolactamlösung zwischen etwa 10 mm/sec und etwa 10 m/sec, vorteilhaft zwischen etwa 10 cm/sec und etwa 3m/sec. Die Strahlerleistung liegt bevorzugt im Bereich zwischen etwa 0,01 Watt/m² und etwa 10 kW/m², bevorzugt 0,1 Watt/m² bis 1500 Watt/m². Eingesetzt werden bevorzugt breitbandige UV-Strahlungsquellen, die maximale Emissionen im Spektralbereich zwischen 150nm und 500nm, bevorzugt zwischen 180nm und 200nm, insbesondere 220nm und 290nm aufweisen. Zur Erzielung eines breitbandigen Spektrums können auch mehrere Strahler unterschiedlicher maximaler Wellenlänge wie beispielsweise mehrere Hochdruckstrahler bzw. Mitteldruckstrahler um 180 nm und 220 nm sowie Niederdruckstrahlern um 260nm miteinander kombiniert werden. Bevorzugt werden Multi Emission-UV-Strahler wie sie beispielweise die Fa. Heraeus im Lieferprogramm hat, die im Wellenlängenbereich zwischen 200 und 600 nm eine Vielzahl von Emissionsmaxima haben.

Die Stufe b) des erfindungsgemäßen Verfahrens wird wenigstens einmal durchgeführt. Sie kann jedoch auch wenigstens zweimal durchgeführt werden. Wie oft Stufe b) des Verfahrens durchgeführt wird, hängt von der verwendeten Vorrichtung, insbesondere dem verwendeten UV-Strahler, ab. Ein UV-Strahler ist in seiner Leistung nur begrenzt regelbar. Beim Aufarbeiten von Caprolactam aus Recyclinganlagen muss mit unterschiedlicher Produktqualität bzw. Reinheit des Caprolactam gerechnet werden. Um den wechselnden Anforderungen gerecht zu werden und nicht zu hoch zu bestrahlen, wählt man bei dem vorgestellten Verfahren eine Kreislaufführung, bei der durch die Zahl der Kreisläufe bei konstanter Strahlerleistung die gewünschte Qualität erzeugt wird. Die gewünschte Qualität wird dadurch gesichert, dass Automatische Analysengeräte vor Eintritt in UV-Strahler und nach Austritt aus dem UV Strahler das Spektrum der wässrigen Caprolactamlösung erfassen und solange an den Strahlern vorbeiführen, bis das als Permanganatzahl definierte Qualitätskriterium erreicht oder unterschritten ist, wie nachstehend detailliert beschrieben.

Die Bestrahlungsmenge bzw. die Anzahl an durchgeführten Stufen b) hängt davon ab, ob das Caprolactam-Rohmaterial bereits nach dem einmaligen Durchführen der Stufe b) die gewünschten Eigenschaften zeigt. Sollte dies nicht der Fall sein, wird Stufe b) sooft, wie erforderlich durchgeführt, um die gewünschten Eigenschaften zu erreichen. Insbesondere hängt die Anzahl an durchgeführten Stufen b) bzw. die Bestrahlungsmenge davon ab, welche Intensität der Analysatordetektor nach dem Durchführen der Stufe b), beispielsweise am Austritt einer Bestrahlungskammer, als Austrittsgröße anzeigt. Ist die Veränderung zwischen einer gemessenen Eingangsgröße vor Stufe b) und der gemessenen Austrittsgröße nach Stufe b) zu gering und ein vorgegebenes Qualtätskriterium nicht erreicht, dann wird ein Ventil für den Weitertransport des Caprolactam-Rohmaterials nicht freigegeben bzw. geöffnet, d.h. das Material zirkuliert bis zum Erreichen seiner Qualitätsvorgaben beliebig oft. Bestimmungsgröße oder Qualitätskriterium sind insbesondere die Lichtdurchlässigkeiten bei 410nm und 420 nm.

Vorzugsweise wird die Bestrahlung durch wenigstens einen UV-Strahler, insbesondere 1 bis 10 UV-Strahlern durchgeführt.

Das in Stufe b) des erfindungsgemäßen Verfahrens bestrahlte Caprolactam-Rohmaterial, insbesondere die Caprolactamlösung, kann bei einer kontinuierlichen Wiederaufbereitung von Polyamiden starken Schwankungen der Eingangsprodukt- und der Rohcaprolactamzusammensetzung unterliegen. Daher ist es bevorzugt, vor dem Durchführen der Stufe b) des erfindungsgemäßen Verfahrens zur Erreichung einer Farbverbesserung, das Caprolactam-Rohmaterial kontinuierlich durch eine lichtdurchlässige Zelle zu führen und kontinuierlich dessen Lichtdurchlässigkeit, bevorzugt in einem Spektralbereich von 200 nm bis 1010 nm, zu erfassen. Die so erfassten Lichtdurchlässigkeitswerte, insbesondere in einem Spektralbereich von 200 nm bis 1010 nm, können einem Mikrocomputer zur Speicherung als Eingangsgröße zugeführt werden.

Bevorzugt kann nach dem Durchführen der Stufe b) des erfindungsgemäßen Verfahrens, insbesondere am Ende eines Reaktors mit dem UV-Strahler, eine weitere Analysenstation angeordnet sein, die die Lichtdurchlässigkeit des mit UV-Strahlung bestrahlten Caprolactam-Materials, bevorzugt im Spektralbereich von 200 nm bis 1010 nm, misst und an einen Mikrocomputer zur Speicherung als Austrittsgröße weitergibt. Damit können über einen Eichbetrieb mit sich kontinuierlich ändernder Zusammensetzung eines wässrigen Caprolactamstromes die Auswirkungen des UV-Reaktors auf die Produktqualität untersucht und gespeichert werden.

Zur Eichung der Messvorrichtung werden Caprolactamlösungen mit einer sehr schlechten Permanganat Farbzahl, d.h. mit hoher Absorption im Bereich der Wellenlängen 410nm und 420 nm, in konventioneller Analysentechnik bestimmt und durch Verdünnung auf Zwischenwerte bzw. niedrigere Lichtabsorption eingestellt und vermessen. Die Kriterien und Standardmessmethoden sind nachstehend beschrieben.

Dieses Caprolactam-Rohmaterial bzw. -Lösungen werden durch Messzellen vor und nach der Stufe b) geführt, die Lichtabsorption an den Regelcomputer weitergeleitet und dort zum Vergleich mit den im Betrieb auftretenden Werten verglichen. Die Kriterien für eine Einstufung als gute Qualität sind von dem Betreiber der Anlage einstellbar und durch Handeingabe am Regelcomputer wandelbar.

Auf die Eichphase kann eine Auswertephase folgen. Falls in der Auswertphase festgestellt wird, dass die Austrittsgröße, d.h. die Lichtdurchlässigkeit des in Stufe b) erhaltenen Caprolactam-Materials, von dem gewünschten Wert abweicht, kann der Durchsatz bei der UV-Bestrahlung in Stufe b) erhöht oder erniedrigt werden. Dies kann vorzugsweise dadurch bewirkt werden, dass ein Förderorgan, das den Durchsatz durch den UV-Reaktor bestimmt, mit einer Messung der Fördermenge und einem phasenanschnittgesteuerten Inverter zur Regelung der Drehzahl und Fördermenge versehen ist.

Insbesondere kann durch den Vergleich der Messung der Lichtdurchlässigkeit des Caprolactam-Rohmaterials vor der Stufe b) und der Lichtdurchlässigkeit des nach der Stufe b) erhaltenen Caprolactam-Materials, die gefundene Schwankung bei konstanter Strahlerleistung durch Anpassung des Durchsatzes oder der Verweilzeit in Stufe b), z.B. im Bestrahlungsraum, ausgeregelt. Insbesondere kann der Durchsatz verlangsamt und/oder die Stufe b) mehrmals durchgeführt werden, wenn die Austrittsgröße nicht den gewünschten Wert hat, wodurch die Produktqualität konstant gehalten werden.

Da es, wie vorstehend beschrieben, bei der kontinuierlichen Wiederaufbereitung von Polyamiden zu starken Schwankungen der Eingangsproduktzusammensetzung und damit zu Schwankungen in der Rohcaprolactamzusammensetzung kommen kann, hat es sich als äußerst vorteilhaft erwiesen, das in die Stufe b) des erfindungsgemäßen Verfahrens zur Erreichung einer Farbverbesserung eintretende Produkt, nämlich das Caprolactam-Rohmaterial, kontinuierlich durch eine lichtdurchlässige Zelle zu führen und durch ständige Verfolgung der Lichtdurchlässigkeit in einem Spektralbereich von 200 nm bis 1010 nm hinsichtlich Schwankungen und Qualität gemäß den vorweg beschriebenen Kriterien zu verfolgen. Es hat sich darüber hinaus als sehr vorteilhaft erwiesen, die Lichtdurchlässigkeitswerte in einem Spektralbereich von 200 nm bis 1010 nm einem Mikrocomputer zur Speicherung als Eingangsgröße zuzuführen. Im dem nächsten, der Qualitätserfassung nachfolgenden Schritt, also in Stufe b) des erfindungsgemäßen Verfahrens wird das Caprolactam-Rohmaterial, insbesondere die Caprolactamlösung, einem UV-Strahler ausgesetzt durch den, Produkte wie Aldehyde, Ketone oder ungesättigte Kohlenwasserstoffe oxidiert werden.

Vorzugsweise kann das in Stufe b) des erfindungsgemäßen Verfahrens zu bestrahlende Caprolactam-Rohmaterial vor dem Durchführen der Stufe b) in wenigstens einem Pufferbehälter gesammelt und, gegebenenfalls nach der Analyse der Lichtdurchlässigkeit in einem Spektralbereich von 200 nm bis 1010 nm und Aufzeichnen der Eingangsgröße, wenigstens einmalig einer UV-Bestrahlung ausgesetzt werden, insbesondere in einem Reaktor mit UV-Strahler, wobei das Caprolactam-Rohmaterial durch wenigstens ein Förderorgan der Bestrahlung in Stufe b) zugeführt wird.

Das nach dem erfindungsgemäßen Verfahren erhaltene Caprolactam ist hochrein und zur Polykondensation von hochwertigem Polyamid 6 geeignet und unterscheidet sich nicht von einem aus industrieller Herstellung stammenden Produkt hinsichtlich der Farbzahl, die durch photometrische Methoden ermittelt wird. In der Qualitätssicherung haben sich die folgenden Methoden zur Caprolactambeurteilung eingebürgert:

In ersten Fall wird eine 1 %-ige wässrige Caprolactamlösung nach Zugabe von 1 Massen% einer 0,01 n KMnO₄-Lösung bei 25 °C nach 250 Sekunden bei der Wellenlänge von 410 nm nach Verfahren Klett-Summerson der 100-fache Wert der Extinktion als Qualitätskriterium bestimmt oder nach 600 Sekunden bei einer Wellenlänge von 420 nm als Permanganatabsorptionszahl . Nach Klett-Summerson sollte der 100-fache Wert der Extinktion 7 nicht überschreiten und bei der Permanganatabsorptionszahl sollte der 100-fache Wert der Extinktion nicht über 5 liegen.

Die zweite Methode bevorzugt die visuelle Beurteilung mit einer Standardlösung. Zu einer 100ml zwischen 1 und 3 Massen% Caprolactam enthaltenden wässrigen Lösung werden bei einer Temperatur von 20°C 1 ml einer 0,01 n Kaliumpermanganatlösung hinzugefügt und die Zeit bis zur Farbgleichheit gegen eine Standardlösung verfolgt. Als Standard sind wässrige Lösungen von Kobaltnitrat/Kobaltchlorid als wässrige Lösungen gemischt mit Kaliumbichromat/Kupfersulfat üblich. Ein Caprolactam gilt als qualitativ gut, wenn eine einprozentige Lösung versetzt mit Kaliumpermanganat gegen eine Lösung aus 1l Wasser enthaltend 2,5g Co(NO₃)₂*6 H₂O und 0,01g K₂CrO₇ gemessen, in 10000 bis 40000 Sekunden gleiche Farbe erreicht.

Im Gegensatz zu einer Caprolactamproduktion aus reinen Rohstoffen, bei der es zu keiner Störung des kontinuierlichen Betriebes durch schwankende Zusammensetzung des eingehenden Produktstromes kommt, können die aus der wechselnden Zusammensetzung des Eingangsmaterials resultierenden Produktqualitätsschwankungen mittels des erfindungsgemäßen Verfahrens ausgeregelt und ausgeglichen werden.

Das erfindungsgemäße Verfahren vermeidet vorteilhaft die zusätzlichen, kosten- und energieverbrauchenden Verfahrensschritte Abscheidung und Abtrennung von Feststoffen dadurch, dass das Caprolactam-Rohmaterial, insbesondere eine Caprolactamlösung, durch Bestrahlung mit UV-Strahlen farblich verbessert wird.

Das erfindungsgemäße Verfahren unter Verwendung einer UV-Bestrahlung, beispielsweise durch Tauchsonden, zeichnet sich gegenüber dem Stand der Technik somit dadurch vorteilhaft aus, dass keine Chemikalien eingesetzt werden müssen. Bei der Zugabe von peroxidischen Chemikalien oder auch beim Permanganat kommt es bei nicht genauer Erfassung der notwendigen Menge im Falle von Überdosierungen zu Störungen nachfolgenden Verarbeitungsprozessen durch deren nicht umgesetzter Anteil. Eine Überdosierung verursacht auch Kosten, die die Wirtschaftlichkeit eines Verfahren mindern. Wird zu wenig eingesetzt, kann die Qualität nicht erreicht werden.

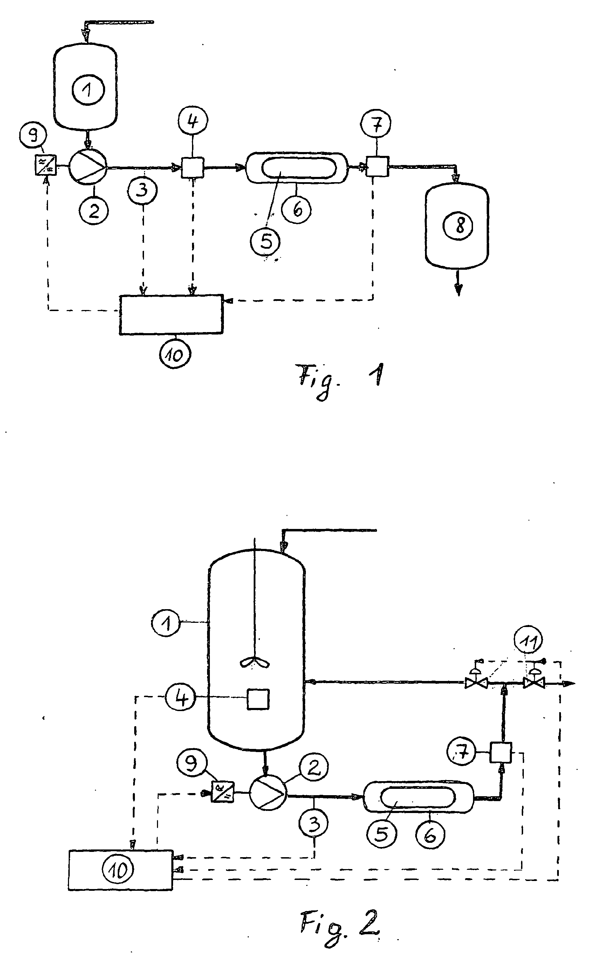

Figuren 1 und 2 zeigen für das Durchführen der Stufe b) des erfindungsgemäßen Verfahrens geeignete Anlagen mit
- 1: Puffer oder Sammelbehälter
- 2: Förderorgan
- 3: Durchflussmessgerät
- 4: Analysengerät Eingangskontrolle, Spektrometer Zeiss MCS 320 + MCS 340
- 5: UV Strahler z.B. Heraeus NIQ 120/80
- 6: Strömungsrohrreaktor
- 7: Analysengerät Ausgangskontrolle, Spektrometer Zeiss MCS 320 + MCS 340
- 8: Puffer oder Sammelbehälter, Ausgang zur Weiterverarbeitung
- 9: Inverter- Phasenanschnittsteuerung zur Förderorgandurchsatzregelung
- 10: Mikrocomputer basierendes Erfassungs-, Auswerte- und Steuerungssystem Steuerung des Durchsatzes abhängig von 4,7
- 11: Teilerventile, Rückführung zum Sammelbehälter und Ausgang zur Weiterverarbeitung

In den Anlagen nach den Figuren 1 oder 2 kann das erfindungsgemäße Verfahren durchgeführt werden. Dazu wird das nach Stufe a) erhaltene Caprolactam-Rohmaterial in einem Puffer 1, in dem zum Messen der Eingangsgröße der Lichtdurchlässigkeit des Caprolactams ein Analysengerät 4 angeordnet ist, gesammelt und sodann mittels eines Förderorgans 2, nach dem ein Durchflußmessgerät 3 angeordnet ist, zum UV-Strahler 5 geleitet, der sich in einem Strömungsrohrreaktor 6 befindet. Die erfindungsgemäß verwendbaren UV-Strahler 5 haben eine fest definierte Strahlungsleistung in Watt/m², die sich nach der benötigten Durchsatzleistung richtet. Ein UV-Strahler kann, nachdem eine Zündung erfolgte, nur in einem engen Bereich geregelt werden. Ändert sich die, bei einer in der Auslegung auf eine gewisse Strahlungsleistung bezogene zu bestrahlende Masse, so tritt nachteilig entweder eine Unter- oder Überdosierung auf. Um dies zu vermeiden, wird vorteilhaft das zu bestrahlende Gut, nämlich das Caprolactam-Rohmaterial, im Pufferbehälter 1 gesammelt und nach der Analyse mittels des Analysegeräts 4 in einem Spektralbereich von 100 nm bis 1010 nm dem Strömungsrohreaktor 6 mit UV-Strahler 5 durch das Förderorgan 2 geregelt zugeführt.

Zum Steuern der Durchsatzgeschwindigkeit durch den Strömungsrohrreaktor 6 ist am Förderorgan 2 eine Inverter-Phasenanschnittssteuerung 9 angeordnet.

Nach dem Strömungsreaktor 6 wird das nach dem Bestrahlen erhaltene Caprolactam durch ein Analysengerät 7 geleitet, in dem die Lichtdurchlässigkeit des erhaltenen Caprolactams als Austrittsgröße gemessen und aufgezeichnet wird. Danach wird das erhaltene Caprolactam in einen weiteren Puffer 8 bis zur weiteren Verwendung und Polymerisation zu Polyamid 6 gelagert, wie in Figur 1 gezeigt. Wie in Figur 2, kann das nach Stufe b) erhaltene Caprolactam auch zu einem Teilerventil 11 geleitet werden und in Abhängigkeit von dem gemessenen Austrittsgröße direkt weiterverarbeitet werden oder in den Puffer 1 zur nochmaligen Bestrahlung zurückgeleitet werden.

Des weiteren umfasst die Anlage einen Mikrocomputer 10, in dem die in den Analysegeräten 4 und 7 gemessenen Eingangs- und Austrittsgrößen gespeichert werden. Der Mikrocomputer 10 ist außerdem mit dem Durchflußmessgerät 3 und der Inverter-Phasenanschnittssteuerung 9 verbunden und regelt in Abhängigkeit von der gemessenen Eingangs- und Austrittsgröße und der Durchflußgeschwindigkeit über die Inverter-Phasenanschnittssteuerung 9 die Durchflußgeschwindigkeit durch den Strömungsreaktor 6.

Die Analysenstation 7, die die Lichtdurchlässigkeit im Spektralbereich von 200 nm bis 1010 nm misst und an den Mikrocomputer 10 zur Speicherung als Austrittsgröße weitergibt, dient dazu, über einen sogenannten Eichbetrieb mit sich kontinuierlich ändernder Zusammensetzung eines wässrigen Caprolactamstromes die Auswirkungen des UV-Reaktors auf die Produktqualität zu untersuchen und zu speichern. Die nach der Eichphase folgende Auswertephase wird unter den Gesichtspunkten geführt, die beste Produktqualität am Reaktoraustritt durch Änderung der Durchsatzleistung bei konstanter Strahlerleistung und sich ändernder Zusammensetzung eines wässrigen Caprolactamstromes zu erreichen. Zu diesem Zweck wird die Pumpe 2, die den Durchsatz durch den UV-Reaktor 6 bestimmt, mit einer Messung des Durchsatzes 3 nach den Stand der Technik und einem phasenanschnittgesteuerten Inverter 9 zur Regelung der Drehzahl und Fördermenge versehen. Durch diese Maßnahmen ist es vorteilhaft möglich, die in der Eintrittsanalysenstation 4 gefundenen Schwankungen bei konstanter Strahlerleistung 5 durch Anpassung des Durchsatzes oder der Verweilzeit im Bestrahlungsraum auszuregeln und die Produktqualität konstant zu halten.

Die Erfindung wird nachstehend an Hand von Beispielen verdeutlicht.

### Beispiel 1

Im Betrieb einer Produktionsanlage zur Aufbereitung von Polyamid 6 Abfällen wie beispielsweise den in verschiedenen Patenten DE 19719734, EP 0681896, US 5598980, DE 2416573, und DE 2507744 beschrieben, erfolgt die Erkennung und Sortierung von Nichtpolyamid 6-Ware, deren Zerkleinerung und die Abscheidung von Schmutz- und Fremdstoffbeigaben. Für die Trennung von einzelnen Komponenten, wie sie beispielsweise bei der Zerkleinerung von Teppichen in Form von Schaumrücken, Polfaser und Träger anfallen, werden bevorzugt mehrstufige Schwimm-/Sinkverfahren benutzt, in deren Folge mittels Zentrifugen und Salzlösungen unterschiedlicher Dichte ein Aufschwimmen der leichteren Fraktion, wie Polypropylengewebe, und ein Absinken von schwererem gefülltem Teppichrücken bewirkt wird. Eine nach diesem Verfahren gewonnene Polyamid 6 Fraktion wird durch Waschen von anhängender Salzfracht befreit und in einem kontinuierlichen Verfahren getrocknet und über einen Extruder der Depolymerisation zugeführt. In der Depolymerisation erfolgt beispielsweise nach den aus DE 887199, EP 0875504, DE 910056, US 4605762, und JP 53-13636 bekannten Verfahren mittels Phosphorsäure eine Spaltung der Polymerketten und eingeleiteter Dampf treibt das entstandene Caprolactam als Wasserdampfgemisch ab.

Nachteilig an den bekannten Verfahren ist es, dass diese kein hochreines Caprolactam, dass zur Herstellung von Polyamid 6 verwendbar ist, liefern. Bei der bisherige Praxis bei der Reinigung von aus Polyamid 6 Teppichen zurück gewonnenem Caprolactam oder wässriger Caprolactamlösung geht man so vor, dass Kaliumpermanganat zugegeben wird. Nachteilig ist, dass das zum Caprolactam zugegebenen Permangant reduziert wird, verbunden mit anschließender Abscheidung von Braunstein.

Wird das Caprolactam oder seine wässriger Lösung einer Vorrichtung nach der in der Erfindung beanspruchten Ausführung in Figur 1 zugeführt, so ergeben sich durch Einsatz des beanspruchten Verfahrens aus den in Tabelle 1 aufgelisteten Qualitätsmerkmale die sowohl produktspezifische als auch wirtschaftliche Vorteile gegenüber anderen Verfahrensweisen. Die Ergebnisse sind Tabelle 1 zu entnehmen.

### Beispiel 1

In einen Behälter 1 wird Rohcaprolactamlösung eingespeist und von der Analyseneinheit 4 vor dem Bestrahlungsapparat das spektrale Verhalten erfasst. Am Boden des Behälters 1 wird von einer dreh- bzw. durchsatzgeregelten Pumpe 2,9 Lösung abgezogen, durchströmt die Durchflussmessstelle 3, wird im Reaktor 6 vom Breitband UV-Strahler 5 mit der festgelegten Leistung beaufschlagt und in der Analyseneinheit 7 auf seine Veränderung kontrolliert. Die Regelventile 11 erhalten von der Mikrocomputer Auswerte- und Steuereinheit 10 die Befehle zur Abgabe bestimmter Mengen in die Weiterverarbeitung und der Rückführung in den Vorratsbehälter 1

**Tabelle 1**

| **Verfahren** | **Oxidationsmittel** | **Permanganatzahl sec** | **Permanganatzahl Schwankungsbreite sec** | **UV-Transmission %** | **UV-Transmission Schwankungsbreite %** | **Kostenvergleich** Investition Verbrauch Energie |
|---|---|---|---|---|---|---|
| Industriell | Unbekannt | >10000 | | >85 | | - |
| Vergleichs- | Permanganat | Min. 5000 | 3000 | Min. 72 | | 100 |
| Verfahren | | Max. 8000 | | Max. 87 | 15 | |
| Vergleichs- | Wasserstoff- | Min. 5500 | 2500 | Min. 80 | | |
| Verfahren | Peroxid | Max. 8000 | | Max. 85 | 12 | 98 |
| Beispiel 1 | UV-Strahler | Min. 10000 | 1000 | Min. 88 | | |
| | | Max.11000 | | Max. 92 | 5 | 90 |

### Beispiel 2

In einen Behälter 1 wird Rohcaprolactamlösung eingespeist und von der Analyseneinheit 4 im Reaktor das spektrale Verhalten erfasst. Am Boden des Behälters 1 wird von einer dreh- bzw. durchsatzgeregelten Pumpe 2,9 Lösung abgezogen, durchströmt die Durchflussmessstelle 3, wird im Reaktor 6 vom Breitband UV-Strahler 5 mit der festgelegten Leistung beaufschlagt und in der Analyseneinheit 7 auf seine Veränderung kontrolliert. Die Regelventile 11 erhalten von der Mikrocomputer Auswerte- und Steuereinheit 10 die Befehle zur Abgabe bestimmter Mengen in die Weiterverarbeitung und der Rückführung in den Vorratsbehälter 1.

## Patentansprüche

1. Verfahren zum Herstellen von Caprolactam aus Polyamid-haltigen Abfällen, umfassend
a) Depolymerisieren der Polyamid-haltigen Abfälle, wodurch ein Caprolactam-Rohmaterial und gegebenenfalls ein Nebenbestandteile bzw. Zuschlagsstoffe enthaltender Strom erhalten wird, und
b) Bestrahlen des Caprolactam-Rohmaterials mit UV-Strahlung;
wobei die Polyamid-haltigen Abfälle, ausgewählt sind aus der Gruppe, bestehend aus gebrauchten, verschmutzten oder dem Recyclingkreislauf entnommenen Polyamid 6 Abfällen, Polyamid-haltigen Formkörpern, Polyamid-haltigen Spritzgussteilen mit Glasfasern und anderen Zuschlägen, Polyamid-haltigen Fasern, Teppichen, Teppichbodenbelägen und anderen Polyamid-haltigen Gegenständen des täglichen Lebens.

2. Verfahren nach Anspruch 1, wobei vor Stufe b) die Lichtdurchlässigkeit des Caprolactam-Rohmaterials gemessen und als Eingangsgröße aufgezeichnet wird.

3. Verfahren nach Anspruch 2, wobei das Caprolactam-Rohmaterial zur Messung der Lichtdurchlässigkeit kontinuierlich durch eine lichtdurchlässige Zelle geführt und durch ständige Verfolgung der Lichtdurchlässigkeit in einem Spektralbereich von 200 nm bis 1010 nm erfasst wird.

4. Verfahren nach einem der Ansprüche 2 oder 3, wobei die Eingangsgröße der Lichtdurchlässigkeit in einem Spektralbereich von 200 nm bis 1010 nm einem Mikrocomputer zur Speicherung zugeführt wird.

5. Verfahren nach einem der vorstehenden Ansprüche, wobei das zu bestrahlende Caprolactam-Rohmaterial vor Durchführen der Stufe b) in wenigstens einem Pufferbehälter gesammelt wird und nach Erfassen der Lichtdurchlässigkeit in einem Spektralbereich von 200 nm bis 1010 nm zum Durchführen der Stufe b) einem Reaktor mit UV-Strahler durch wenigstens ein Förderorgan der Bestrahlung zugeführt wird.

6. Verfahren nach einem der vorstehenden Ansprüche, wobei die Bestrahlung in Stufe b) durch wenigstens einen UV-Strahler erfolgt.

7. Verfahren nach Anspruch 6, wobei die Stufe b) in dem wenigsten einem Reaktor mit wenigstens einem umströmten UV-Strahler durchgeführt wird.

8. Verfahren nach einem der vorstehenden Ansprüche, wobei nach Stufe b) die Lichtdurchlässigkeit des nach Stufe b) erhaltenen Caprolactams gemessen und aufgezeichnet wird.

9. Verfahren nach Anspruch 8, wobei die Messung der Lichtdurchlässigkeit im Spektralbereich von 200 nm bis 1010 nm durchgeführt und an einen Mikrocomputer zur Speicherung als Austrittsgröße weiter gegeben wird.

10. Verfahren nach einem der vorstehenden Ansprüche, wobei über einen Eichbetrieb mit sich kontinuierlich ändernder Zusammensetzung eines wässrigen Caprolactamstromes die Auswirkungen des UV-Reaktors auf die Produktqualität untersucht und gespeichert wird.

11. Verfahren nach Anspruch 10, wobei auf die Eichphase eine Auswertephase folgt.

## Claims

1. A method for the production of caprolactam from waste containing polyamide, comprising
a) depolymerisation of the waste containing polyamide, whereby a caprolactam raw material and, optionally, a flow containing secondary constituents or additives is obtained, and
b) irradiation of the caprolactam raw material with UV radiation;
wherein the waste containing polyamide is selected from the group consisting of used, contaminated polyamide 6 waste or polyamide 6 waste obtained from the recycling circulation, moulded parts containing polyamide, injection moulded parts with glass fibres and other additives containing polyamide and fibres, carpets, carpet floor coverings containing polyamide and other objects containing polyamide from daily life.

2. The method according to Claim 1, wherein before stage b) the light transmission of the caprolactam raw material is measured and recorded as the input variable.

3. The method according to Claim 2, wherein for the measurement of the light transmission the caprolactam raw material is continuously passed through a light-transparent cell and is measured in a spectral range from 200 nm to 1010 nm by continual tracking of the light transmission.

4. The method according to one of the Claims 2 or 3, wherein the input variable of the light transmission in a spectral range from 200 nm to 1010 nm is passed to a microcomputer for storage.

5. The method according to one of the previous claims, wherein the caprolactam raw material to be irradiated is collected in at least one buffer vessel before carrying out stage b) and after acquisition of the light transmission in a spectral range from 200 nm to 1010 nm is fed for carrying out stage b) to a reactor with UV radiator by at least one transport mechanism for irradiation.

6. The method according to one of the previous claims, wherein the irradiation in stage b) occurs by at least one UV radiator.

7. The method according to Claim 6, wherein stage b) is carried out in the at least one reactor with at least one UV radiator enclosed by flow.

8. The method according to one of the previous claims, wherein after stage b) the light transmission of the caprolactam obtained after stage b) is measured and recorded.

9. Method according to Claim 8, wherein the measurement of the light transmission is carried out in the spectral range from 200 nm to 1010 nm and is passed to a microcomputer for saving as the output variable.

10. The method according to one of the previous claims, wherein the effects of the UV reactor on the product quality are examined by means of a calibration mode with continually varying composition of an aqueous caprolactam solution and are stored.

11. The method according to Claim 10, wherein an evaluation phase follows the calibration phase.

## Revendications

1. Procédé pour la préparation de caprolactame à partir de déchets contenant du polyamide comprenant
a) la dépolymérisation des déchets contenant du polyamide, sur quoi l'on obtient un matériau brut de caprolactame et éventuellement un courant contenant des constituants secondaires respectivement des agrégats, et
b) l'irradiation du matériau brut de caprolactame avec un rayonnement UV ; les déchets contenant du polyamide étant choisis parmi des déchets de polyamide 6 usés, salis ou prélevés du recyclage, des corps moulés contenant du polyamide, des pièces moulées par injection contenant du polyamide avec des fibres de verre et d'autres additifs, des fibres contenant du polyamide, des tapis, des revêtements de sol de tapis et d'autres objets contenant du polyamide de la vie quotidienne.

2. Procédé selon la revendication 1, dans lequel on mesure avant l'étape b) la transmission de la lumière du matériau brut de caprolactame et on la caractérise comme grandeur de départ.

3. Procédé selon la revendication 2, dans lequel on introduit le matériau brut de caprolactame pour la mesure de la transmission de la lumière en continu à travers une cellule transparente et on enregistre par poursuite continue de la transmission de la lumière dans un domaine spectral de 200 nm à 1010 nm.

4. Procédé selon l'une quelconque des revendications 2 ou 3, dans lequel on introduit les grandeurs de départ de la transmission de la lumière dans un domaine spectral de 200 nm à 1010 nm dans un micro-ordinateur pour un stockage.

5. Procédé selon l'une quelconque des revendications précédentes, dans lequel le matériau brut de caprolactame à irradier est recueilli avant la réalisation de l'étape b) dans au moins un récipient tampon et est, après l'enregistrement de la transmission de la lumière dans un domaine spectrale de 200 nm à 1010 nm, introduit pour la réalisation de l'étape b) dans un réacteur avec un dispositif d'irradiation UV par l'intermédiaire d'au moins un organe de convoyage de l'irradiation.

6. Procédé selon l'une quelconque des revendications précédentes, dans lequel l'irradiation dans l'étape b) est réalisée par au moins un dispositif d'irradiation UV.

7. Procédé selon la revendication 6, dans lequel on réalise l'étape b) dans le au moins un réacteur avec au moins un dispositif d'irradiation UV parcouru par un courant.

8. Procédé selon l'une quelconque des revendications précédentes, dans lequel on mesure après l'étape b) la transmission de la lumière du caprolactame obtenu après l'étape b) et on la caractérise.

9. Procédé selon la revendication 8, dans lequel on réalise la mesure de la transmission de la lumière dans le domaine spectral de 200 nm à 1010 nm et on l'introduit à nouveau dans un micro-ordinateur pour le stockage comme grandeur de sortie.

10. Procédé selon l'une quelconque des revendications précédentes, dans lequel on étudie par l'intermédiaire d'un système d'étalonnage avec une composition d'un courant aqueux de caprolactame variant en continu les effets du réacteur UV sur la qualité du produit et on les stocke.

11. Procédé selon la revendication 10, dans lequel la phase d'étalonnage est suivie par une phase d'exploitation.
